# EUROPÄISCHE PATENTSCHRIFT

(11) **EP 3 363 541 B1**
(45) Veröffentlichungstag und Bekanntmachung des Hinweises auf die Patenterteilung: **17.06.2020**
(21) Anmeldenummer: 17156764.7
(22) Anmeldetag: 17.02.2017
(51) Int. Cl.: B01J 31/02, B01J 31/22, C07C 45/33

(54) **KATALYSATORMISCHUNG UND VERFAHREN ZUR SELEKTIVEN OXIDATION VON ORGANISCHEN SUBSTRATEN MIT SAUERSTOFF**
CATALYST MIXTUR AND PROCESS FOR THE SELECTIVE OXIDATION OF ORGANIC SUBSTRATES WITH OXYGEN
MÉLANGE DE CATALYSEUR ET PROCÉDÉ D'OXYDATION SÉLECTIVE DE SUBSTRATS ORGANIQUES AVEC OXYGÈNE

(43) Veröffentlichungstag der Anmeldung: 22.08.2018
(73) Patentinhaber: Justus-Liebig-Universität Gießen, 35390 Gießen (DE)
(72) Erfinder: SCHINDLER, Siegfried, 35392 Gießen (DE); MISKA, Andreas, 35398 Gießen (DE); GAWLIG, Christopher, 35390 Gießen (DE)
(74) Vertreter: Stumpf, Peter

(56) Entgegenhaltungen:
- SABINE LÖW ET AL: "Reactions of Copper(II) Chloride in Solution: Facile Formation of Tetranuclear Copper Clusters and Other Complexes That Are Relevant in Catalytic Redox Processes", CHEMISTRY - A EUROPEAN JOURNAL, Bd. 19, Nr. 17, 22. April 2013 (2013-04-22), Seiten 5342-5351, XP055368762, ISSN: 0947-6539, DOI: 10.1002/chem.201203848
- KNOPS-GERRITS P-P ET AL: "Alkane oxidation by dinuclear iron complexes in hexagonal mesoporous solids", MICROPOROUS AND MESOPOROUS MATERIALS, ELSEVIER, AMSTERDAM, NL, Bd. 21, Nr. 4-6, 1. Mai 1998 (1998-05-01), Seiten 475-486, XP004128397, ISSN: 1387-1811, DOI: 10.1016/S1387-1811(98)00132-2
- KORTNEY L. KLINKEL ET AL: "Rapid phosphorus triester hydrolysis catalyzed by bimetallic tetrabenzimidazole complexes", CHEMICAL COMMUNICATIONS - CHEMCOM., Nr. 27, 1. Januar 2006 (2006-01-01), Seite 2919, XP055369187, ISSN: 1359-7345, DOI: 10.1039/b602769e

## Beschreibung

Der Gegenstand der Erfindung umfasst ein Verfahren zur selektiven Oxidation von organischen Substraten, insbesondere der Oxidation von Alkanen zu Ketonen oder Aldehyden unter Verwendung von Sauerstoff als Oxidationsmittel sowie Katalysatormischungen zur Durchführung des Verfahrens.

### Stand der Technik

Die Oxidation von organischen Substraten ist ein wichtiger Prozeßschritt in zahlreichen Chemischen Produktionsverfahren. Als organische Substrate werden in diesem Zusammenhang alle chemischen Verbindungen verstanden, die dem Fachmann als sogenannte organische Verbindungen geläufig sind. Diese können unterschiedlichster Struktur sein und auch bereits eines oder mehrere Sauerstoffatome oder andere sogenannte Heteroatome bzw. funktionelle Gruppen enthalten und weisen als Gemeinsamkeit auf, ein oder mehrere Kohlenstoffatome zu enthalten.

Im Stand der Technik sind zahllose verschiedene Oxidationsverfahren für unterschiedlichste organische Substrate bekannt. Insbesondere im Bereich der Herstellung von Aldehyden und Ketonen durch direkte Oxidation besteht jedoch das Problem, dass alle bekannten Oxidationsmittel entweder sehr unselektiv wirken, oder sehr teuer und damit für einen großtechnischen Einsatz ungeeignet sind. Das heißt, man erhält im Zuge der Oxidation (beispielsweise mit Luftsauerstoff) neben dem gewünschten Zielprodukt zahlreiche unerwünschte Nebenprodukte, so dass das erhaltene Produktgemisch mit großem Aufwand und Kosten in die einzelnen Bestandteile aufgetrennt werden muss. Der großtechnische Einsatz eines selektiven Oxidationsmittels zur Herstellung von Aldehyden oder Ketonen ist aus Kostengründen nicht möglich.

Aldehyde und Ketone sind jedoch oftmals wichtige Basischemikalien, die in großen Mengen benötigt werden, beispielsweise Cyclohexanon als Ausgangsprodukt für Caprolactam, das wiederum ein äußerst wichtiger Ausgangsstoff für die Produktion von Polyamid-6 ist und weltweit in großem Maßstabe produziert wird.

Die EP 2 818 459 A1 offenbart beispielsweise die Herstellung von Cyclohexanon in einem mehrstufigen Prozeß, wobei der erste Schritt darin besteht, Cyclohexan nicht-katalytisch mit Sauerstoff zu Cyclohexyl-hydroperoxid zu oxidieren, wobei ein sehr komplexes und schwer auftrennbares intermediäres Produktgemisch erhalten wird. Dieses Produktgemisch wird in einem weiteren Schritt zunächst katalytisch mit Hilfe eines übergangsmetallhaltigen Katalysators zersetzt, bevor die daraus resultierende endgültige Produktmischung rektifiziert werden kann. Dieses Verfahren und die Produktaufbereitung sind sehr langwierig und aufwendig. Zudem handelt es sich bei den im Stand der Technik verwendeten Katalysatoren um solche, die gesundheitlich bedenkliche Schmermetalle wie beispielsweise Kobalt oder Chrom enthalten; es sind keine Kombinationskatalysatoren im Sinne der Erfindung, und sie werden auch nicht im eigentlichen Oxidationsprozeß mit Sauerstoff eingesetzt.

Andere Verfahren, wie sie etwa in der EP 2 982 663 A1 offenbart sind, gehen von Phenol aus, hydrieren dieses unter Verwendung von Palladiumkatalysatoren zu Cyclohexanol und dehydrieren dieses zu Cyclohexanon mit Hilfe von Kupferoxid/Zinkoxid-Katalysatoren (vgl. US 4,918,239 A). Diese Verfahren sind ebenfalls sehr langwierig und erfordern eine aufwendige Produktreinigung. Bei den hier verwendeten Kupferoxid/Zinkoxid-Katalysatoren handelt es sich ebenfalls nicht um Kombinations-Katalysatoren im Sinne der vorliegenden Erfindung, da es sich um oxidische/keramische Festkörper, also Heterogenkatalysatoren handelt. Bei diesen ist es bekannt, dass mehrere, unter Umständen verschiedene, Metallzentren in dem Festkörper insgesamt den Katalysator bilden.

Bei der hier kurz diskutierten Herstellung von Cyclohexanon handelt es sich nur um ein besonders eminentes Beispiel für die im Stand der Technik bestehenden Probleme bei der Oxidation von Organischen Substraten mit Sauerstoff. Das vorstehend gesagte gilt in analoger Form für zahlreiche weitere Oxidationsreaktionen.

Beispielsweise wird in der Publikation von S. Löw et al. (Chem. Eur. J. 2013, 19, 5342ff.) die Oxidation von Cyclohexan zu Cyclohexanon und von n-Hexan zu Hexanol in Gegenwart eines Katalysators beschrieben, der kupferhaltige Clusterverbindungen enthält. Die betreffenden Clusterverbindungen weisen als Liganden Benzylamin bzw. Benzyl-(2-benzylimino-1-methyl-propyliden)-amin auf, und unterscheiden sich deutlich von den erfindungsgemäßen Metallkomplexen der allgemeinen Formel (I).

In der Publikation von K. L. Klinkel et al. (Chem. Commun., 2006, S. 2919ff.) werden Kobaltkomplexe mit HPTB als Liganden beschrieben, sowie ihre Verwendung als Katalysatoren in der Hydrolyse von Phosphorsäureestern. Hinweise auf die Verwendung von Metallkomplexen mit HPTB-Liganden als Katalysatoren für Oxidationen finden sich darin nicht.

Keines der beiden letztgenannten Dokumente beschreibt sowohl Metallkomplexe der Formel I als auch Metallkomplexe der Formel II als Katalysatoren.

Es besteht daher dringender Bedarf daran, die Oxidation von Organischen Substraten mit Sauerstoff selektiver, effizienter und einfacher durchführbar zu gestalten.

### Aufgabe

Aufgabe der vorliegenden Erfindung ist es somit, ein Verfahren zur Verfügung zu stellen, das die vorstehend beschriebenen Nachteile des Standes der Technik beseitigt, das heißt, die Oxidation von Alkanen mit Sauerstoff selektiver, effizienter und einfacher durchführbar zu gestalten. Weiterhin ist es Aufgabe der vorliegenden Erfindung, mindestens eine Katalysatormischung bereitzustellen, mit der das erfindungsgemäße Verfahren durchgeführt wird.

### Lösung der Aufgabe

Diese Aufgabe wird erfindungsgemäß gelöst, indem die Oxidation des Alkans mittels Sauerstoff durch den gemeinsamen Einsatz mindestens zweier Katalysatoren entsprechend dem kennzeichnenden Teil des Anspruchs 1 durchgeführt wird. Die abhängigen Ansprüche 2 bis 15 stellen weitere vorteilhafte Ausgestaltungen des erfindungsgemäßen Verfahrens dar. Anspruch 16 offenbart erfindungsgemäße Katalysatormischungen bzw. Kombinationskatalysatoren. Die Begriffe "Katalysatormischung" und "Kombinationskatalysator" werden im Rahmen dieser Offenbarung synonym verwendet und bezeichnen jeweils eine Mischung von mindestens zwei Metallkomplexverbindungen, die gemeinsam die Oxidation von Alkanen mit Sauerstoff katalysieren.

Dieser überraschende Befund, daß die Verwendung von mindestens zwei Metallkomplexverbindungen die Oxidation von Alkanen mittels Sauerstoff katalysiert, bildet die Grundlage des erfindungsgemäßen Verfahrens.

Das Verfahren ist in allgemeiner Form anhand des Beispiels Cyclohexan/Cyclohexanon in Schema (A) dargestellt.

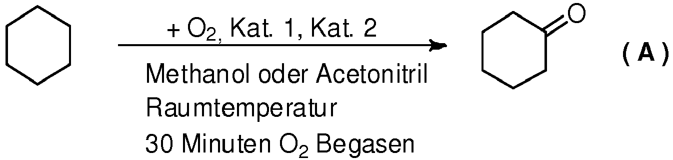

Mit "Kat. 1" und "Kat. 2" werden hierbei zwei verschiedene Metallkomplexverbindungen ("Katalysator 1", "Katalysator 2") bezeichnet, die beide an dem Oxidationsprozeß des Eduktes (Bsp. Cyclohexan) beteiligt sind, wobei die zeitliche Abfolge der Zugabe der beiden Katalysatoren sowie die genaue Art der Zusammenführung der Katalysatoren in der Reaktionsmischung relevant ist, je nach Art des Substrates, Variante des jeweiligen Katalysators oder der für das Gesamtsystem angestrebten Reaktivität bzw. Spezifität.

Kat. 1 und Kat. 2 bilden somit eine Katalysatormischung bzw. einen Kombinationskatalysator, wobei der genaue Mechanismus, wie die beiden Metallkomplexe miteinander und mit dem Edukt interagieren bis dato noch nicht bekannt ist. Das erfindungsgemäße Verfahren zur Oxidation von Organischen Substraten umfasst somit die folgenden Varianten der sequenziellen Zusammenführung von Kat. 1, Kat. 2 und Edukt sowie Oxidationsmittel:
a) Kat. 1 wird zunächst mit dem Edukt zusammengeführt, anschließend wird Kat. 2 hinzugefügt,
b) Kat. 2 wird zunächst mit dem Edukt zusammengeführt, anschließend wird Kat. 1 hinzugefügt,
c) Kat. 1 und Kat. 2 werden zunächst miteinander vermischt, anschließend wird das Edukt hinzugefügt.

Vor jeder Edukt bzw. Teil-Katalysatorzugabe (Kat. 1 oder Kat. 2) sowie während der gesamten Reaktionsdauer können - je nach Bedarf - unterschiedlichste Aktivierungsschritte durchgeführt werden wie beispielsweise die Zuleitung von Gasen (auch Sauerstoff), Bestrahlung mit Ultraschall, sichtbarem Licht oder UV-Strahlung. Die Einspeisung von thermischer Energie kann auf jede im Stand der Technik bekannte Art und Weise erfolgen, ohne den Umfang der Erfindung oder Ihrer Äquivalente zu verlassen.

Bei den erfindungsgemäßen Katalysatormischungen handelt es sich nicht um Mischkatalysatoren, wie sie im Stand der Technik bekannt sind, beispielsweise in Form von Heterogenkatalysatoren auf Basis von Metall-Mischoxiden bzw. dotierten Metalloxiden. Es handelt sich auch nicht um die im Stand der Technik bekannten mehrkernigen Homogenkatalysatoren (mehrkernige MetallKomplexverbindungen). Vielmehr können auch mit im Stand der Technik bekannten ein- oder mehrkernigen homogenen Metallkomplexen erfindungsgemäße Katalysatormischungen hergestellt, bzw. das erfindungsgemäße Verfahren durchgeführt werden.

Ohne an eine Theorie gebunden zu sein kann zum gegenwärtigen Zeitpunkt und unter Berücksichtigung des aktuellen Standes der Technik davon ausgegangen werden, dass sich im Zuge der Vermischung der beiden Katalysatoren Kat. 1 und Kat. 2 aus ihnen kein neuer Komplex als eigentlich wirksamer Katalysator ausbildet. Das heißt, es ist nicht davon auszugehen, daß sich zwischen den beiden Katalysatoren Kat. 1 und Kat. 2 im Zuge der Bildung der erfindungsgemäßen Katalysatormischung bzw. im Verlaufe der Katalyse eine chemische Bindung ausbildet. Als relativ sicher kann jedoch angesehen werden, dass der zweikernige Metallkomplex (Kat. 1) als sogenanntes Peroxid-Shuttle dient. Sauerstoff wird dabei über diesen Komplex zum Peroxid umgewandelt und auf den vierkernigen Metallkomplex (Kat. 2) übertragen.

Die hier offenbarten Katalysatormischungen umfassen erfindungsgemäß Metallkomplexverbindungen der nachfolgend beschriebenen Arten.

Bei Katalysator 1 handelt es sich um Metallkomplexe der allgemeinen Formel (I) und bei Katalysator 2 um Metallkomplexe der allgemeinen Formel (II), wobei letztere Metallkomplexe auch als "µ4-Oxido-Cluster" bezeichnet werden.

[M₂(R-HPTB)X₃] (I) [M'₄OX₆L] (II)

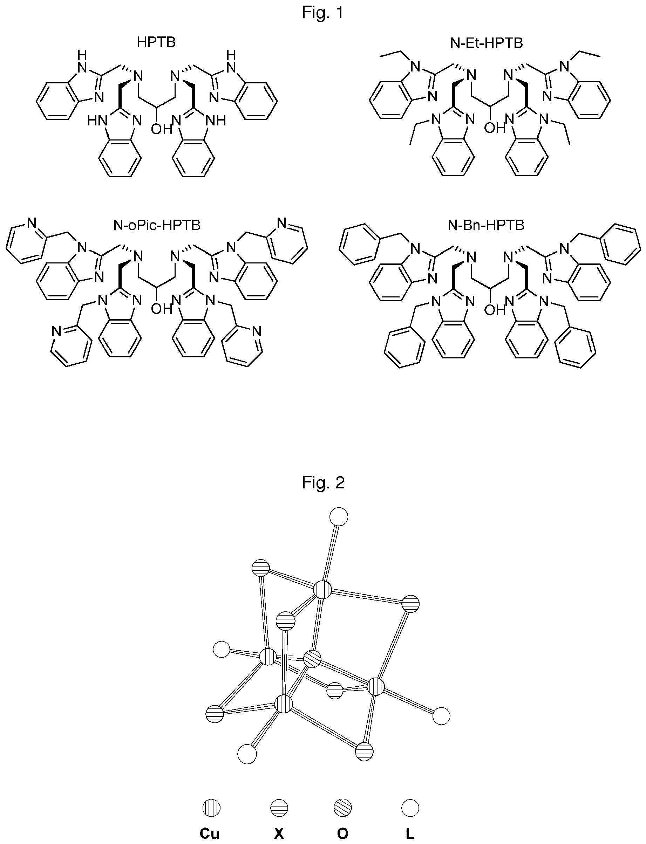

R-HPTB bezeichnet hier mehrzähnige N,N,N',N'-tetrakis(2-R-benzimidazolylmethyl)2-hydroxy-1,3-diaminopropan-Liganden wie sie in Formel (III) beispielhaft in allgemeiner Form anhand eines Eisenkomplexes dargestellt sind. Bei X handelt es sich um Halogene, bevorzugt Chlor oder Brom. Weitere Beispiele für erfindungsgemäße Liganden sind in Fig. 1 gezeigt, wobei diese Zusammenstellung nicht einschränkend zu verstehen ist; dem Fachmann sind zahlreiche weitere organische Reste R bekannt, mit denen HPTB-Liganden versehen werden können, und die daher vom Umfang der Erfindung sowie ihrer Äquivalente mit umfasst werden. Bei den Metallen M und M' handelt es sich bevorzugt um die Metalle Fe, Co, Ni (M) sowie Cu, Ag, Au (M'), wobei auch diese Aufzählungen nicht einschränkend zu verstehen sind.

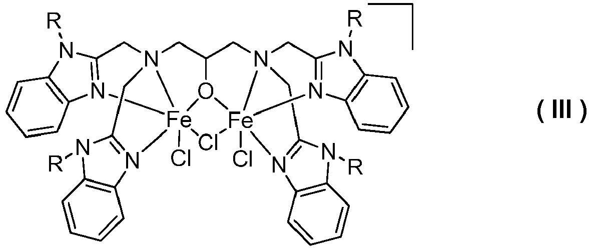

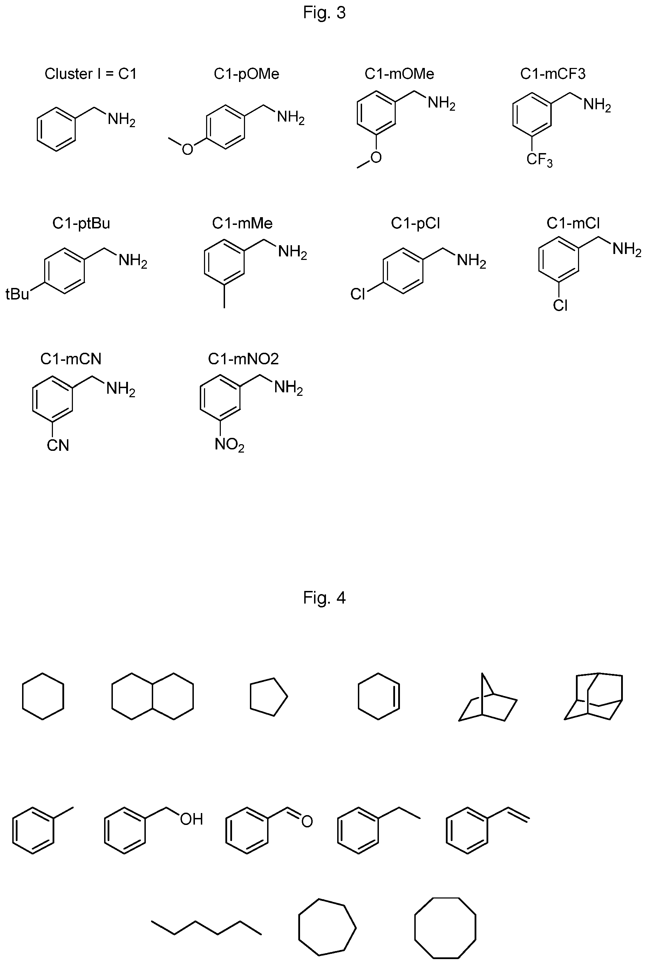

Katalysator 2 weist die in Fig. 2 gezeigte allgemeine Struktur auf, wobei es sich bei den Liganden L um stickstoffhaltige Liganden handelt, wie sie beispielhaft in Fig. 3 gezeigt sind.

Dem Fachmann ist bekannt, dass Oxidationsverfahren von Alkanen bzw. Alkylseitenketten von aromatischen Verbindungen grundsätzlich allgemein anwendbar sind. Es ist daher ohne weiteres ersichtlich, dass das erfindungsgemäße Verfahren in der hier beschriebenen Form, bzw. in äquivalenter Durchführung nicht nur für die Oxidation von Cyclohexan zu Cyclohexanon durchführbar ist, sondern generell für die Oxidation von Alkanen, Cycloalkanen sowie aromatischen Verbindungen mit Alkylseitenketten und Käfigverbindungen verwendet werden kann. Beispiele von Edukten dafür sind in Fig. 4 zusammengestellt, wobei auch diese Zusammenstellung erfindungsgemäßer Edukte nicht einschränkend zu verstehen ist.

Weiterhin ist dem Fachmann bekannt, dass die genauen Reaktionsbedingungen von Oxidationsverfahren je nach konkretem Edukt variiert werden müssen, um für das jeweilige konkret gewünschte Produkt die bestmöglichen Ausbeuten bei geringstmöglichem Aufwand zu erreichen. Die Reaktionsbedingungen des erfindungsgemäßen Verfahrens können daher hinsichtlich der verwendeten Katalysatoren (Kat. 1, Kat. 2) sowie deren Konzentrationen, eingesetztem Edukt, gewünschtem Produkt, Druck, Temperatur und Oxidationsmittel in weiten Grenzen variiert werden, ohne den Umfang der Erfindung sowie Ihrer Äquivalente zu verlassen.

Der Sauerstoff kann in dem erfindungsgemäßen Verfahren in einem sehr weiten Konzentrationsbereich zugeführt werden, der sich von 100% (reiner Sauerstoff) bis auf unter 10% erstreckt, je nach Reaktivität des Eduktes bzw. des angestrebten Oxidationspotentials im Verfahren. Üblicherweise wird er gasförmig zugeführt. Am allermeisten bevorzugt sind dabei Konzentrationen um 20 Prozent (20% bis 25%) und nahe 100 Prozent, da in diesen Fällen Luft ohne oder mit nur geringer Aufreinigung verwendet werden kann (ca. 21% Sauerstoff), bzw. Sauerstoff in Reinform (100% Sauerstoff), der die kostengünstigste Handelsform gasförmigen Sauerstoffs darstellt. Beliebige andere Konzentrationen, etwa jeweils ca. 10%, 30%, 40%, 50%, 60%, 70%, 80% oder 90% oder Zwischenwerte der genannten Konzentrationen können erfindungsgemäß verwendet werden. Diese Konzentrationen bzw. Konzentrationsbereiche müssen jedoch unter Verwendung geeigneter Inertgase zunächst hergestellt werden, was das Verfahren etwas kostenaufwendiger werden läßt. Geeignete Inertgase sind alle nicht-oxidierend wirkenden Gase, wie zum Beispiel Stickstoff, Helium, Neon, Argon und Kohlendioxid. Auch diese Auflistung ist nicht einschränkend zu verstehen.

Äußerst wichtig, aber auch sehr stark vom jeweiligen Edukt abhängig, ist die korrekte (auch zeitliche) Reihenfolge der Zugabe der Reagenzien sowie die Temperatur- und Druckführung der Umsetzung. Diesbezüglich erstreckt sich das erfindungsgemäße Verfahren daher über besonders weite Bereiche möglicher Werte dieser Parameter.

Ein weiterer für die Durchführung des erfindungsgemäßen Verfahrens sehr wichtiger Parameter ist das Mischungsverhältnis der mindestens zwei Metallkomplexverbindungen Kat. 1 und Kat. 2, die die Katalysatormischung bilden. Je nach gewünschter Aktivität der Katalysatormischung oder Reaktivität des Eduktes ist es erforderlich, die mindestens zwei Metallkomplexverbindungen der Katalysatormischung in unterschiedlichem Verhältnis zueinander einzusetzen. Naturgemäß kann dieses Mischungsverhältnis in weiten Bereichen variiert werden, etwa von 1:100 bis 100:1 (Kat. 1 : Kat. 2) ohne den Umfang der Erfindung oder ihrer Äquivalente zu verlassen. Im Falle der bislang beispielhaft genauer untersuchten Eisen- und Kupferkomplexe hat sich ein Verhältnis von 100:25 als vorteilhaft erwiesen.

Bei Bedarf und je nach Art des verwendeten Katalysators 1 oder Katalysators 2 kann dieser jeweils auch vor der Verwendung, bzw. der Kombination mit dem anderen Katalysator zuvor unter Schutzgas für die Verwendung vorbereitet, beispielsweise aktiviert, werden.

Zudem ist die Durchführung des erfindungsgemäßen Verfahrens nicht an einen bestimmten Reaktortyp gebunden oder auf den Labormaßstab begrenzt. Zusammenfassend kann der Gegenstand der Erfindung wie folgt beschrieben werden:
Die Erfindung umfasst ein Verfahren zur Oxidation von organischen Substraten unter Verwendung von Sauerstoff als Oxidationsmittel, wobei mindestens zwei unabhängig voneinander ausgewählte Katalysatoren gemäß den allgemeinen Formeln [M₂(R-HPTB)X₃] (Katalysator 1) und [M'₄OX₆L] (Katalysator 2) nacheinander oder gleichzeitig verwendet werden, um die Oxidationsreaktion zu katalysieren. Bei den Metallatomen M handelt es sich um zwei jeweils unabhängig voneinander ausgewählte Metalle aus der Liste umfassend Fe, Co, Ni. Bei den Metallatomen M' handelt es sich um Atome eines Metalls, ausgewählt aus der Liste umfassend Cu, Ag, Au. Bei den Resten R handelt es sich um unabhängig voneinander aus der Liste umfassend H, Methyl, Ethyl, Propyl, Benzyl, 2-Methyl-Pyridyl ausgewählte Reste. Bei X handelt es sich um Halogenatome, unabhängig voneinander ausgewählt aus der Liste umfassend Cl, Br. Bei den Liganden L handelt es sich um stickstoffhaltige Liganden, unabhängig voneinander ausgewählt aus der Liste umfassend aliphatische Amine, aromatische Amine, Methylaminobenzol sowie substituierte Methylaminobenzole. Das Verfahren wird je nach Reaktivität des Edukts sowie von Katalysator 1 und Katalysator 2 in Gegenwart oder in Abwesenheit eines Lösungsmittels oder Lösungsmittelgemisches durchgeführt.

Weiterhin umfasst die Erfindung ein Verfahren wie vorstehend beschrieben, wobei entweder a) Katalysator 1 zunächst mit dem zu oxidierenden organischen Substrat umgesetzt und anschließend Katalysator 2 hinzugefügt wird, oder b) Katalysator 2 zunächst mit dem zu oxidierenden organischen Substrat umgesetzt und anschließend Katalysator 1 hinzugefügt wird, oder c) zunächst Katalysator 1 und Katalysator 2 miteinander vermischt und anschließend das zu oxidierende organische Substrat hinzugefügt und umgesetzt wird.

Weiterhin umfasst die Erfindung ein Verfahren wie im vorausgegangenen Absatz beschrieben, wobei zwischen den Zugaben von Katalysator 1, Katalysator 2 sowie dem zu oxidierenden organischen Substrat mindestens ein Aktivierungsschritt zwischengeschaltet wird, um die Reaktivität der Reaktionsmischung in der jeweils vorliegenden Zusammensetzung zu verbessern. Dabei handelt es sich bei dem Aktivierungsschritt um Rühren und/oder Heizen und/oder Kühlen und/oder Begasung mit einem Gas und/oder Belichtung und/oder Beschallung mittels Ultraschall und/oder Zugabe weiterer Reagenzien.

Weiterhin umfasst die Erfindung ein Verfahren wie vorstehend beschrieben, wobei das Oxidationsmittel Sauerstoff gasförmig in einer Konzentration von 1% bis 100% zugeführt wird, oder das Oxidationsmittel Sauerstoff in einer Konzentration von 20% bis 25% zugeführt wird, oder das Oxidationsmittel Sauerstoff in einer Konzentration von 80% bis 100% zugeführt wird.

Weiterhin umfasst die Erfindung ein Verfahren wie vorstehend beschrieben, wobei das Mischungsverhältnis von Katalysator 1 zu Katalysator 2 im Bereich von 1:100 bis 100:1 liegt, bzw. das Mischungsverhältnis von Katalysator 1 zu Katalysator 2 im Bereich von 100:20 bis 100:30 liegt. Letzterer Bereich ist besonders bevorzugt, wenn es sich bei Katalysator 1 um Eisenkomplexverbindungen handelt und bei Katalysator 2 um Kupfer-Komplexverbindungen.

Weiterhin umfasst die Erfindung ein Verfahren wie vorstehend beschrieben, wobei das Verfahren bei einem Absolutdruck von 10² Pascal bis 10⁸ Pascal durchgeführt wird. Besonders bevorzugt ist die Durchführung bei einem Druck zwischen 10⁴ Pascal und 10⁶ Pascal, also in der Nähe von Normaldruck (1013 hPa).

Weiterhin umfasst die Erfindung ein Verfahren wie vorstehend beschrieben, wobei das Verfahren bei einer Temperatur im Bereich von -80 °C bis +300 °C durchgeführt wird. Besonders bevorzugt ist die Durchführung des Verfahrens zwischen -10 °C und +100 °C, also in der Nähe von Raumtemperatur, also ca. 25 °C, da dann nur wenig oder keine Energie für die Temperierung aufgewendet werden muß.

Weiterhin umfasst die Erfindung ein Verfahren wie vorstehend beschrieben, wobei das Verfahren unter Bestrahlung mit elektromagnetischen Wellen der Wellenlängenbereiche des UV-, des sichtbaren- oder des Mikrowellenbereichs durchgeführt wird.

Weiterhin umfasst die Erfindung ein Verfahren wie vorstehend beschrieben, wobei das Verfahren in der Gasphase durchgeführt wird. Dabei ist Katalysator 1 und Katalysator 2 auf einer geeigneten Oberfläche immobilisiert und sowohl das Oxidationsmittel als auch das zu oxidierende organische Substrat werden gasförmig mit Katalysator 1 und Katalysator 2 in Kontakt gebracht und umgesetzt.

Weiterhin umfasst die Erfindung ein Verfahren wie vorstehend beschrieben, wobei das zu oxidierende organische Substrat ausgewählt ist aus der Liste umfassend Cyclopentan, Cyclohexan, Cycloheptan, Cyclooctan, Cycloalkane, Methan, Ethan, Propan, Butan, Pentan, Hexan, Heptan, Octan, Alkane, Toluol, o-Xylol, m-Xylol, p-Xylol, Ethylbenzol, Propylbenzol, aromatische Verbindungen mit Alkylseitenketten, Adamantan, Kuban, Käfigverbindungen.

Weiterhin umfasst die Erfindung eine Katalysatormischung zur Oxidation von organischen Substraten unter Verwendung von Sauerstoff als Oxidationsmittel, die mindestens zwei unabhängig voneinander ausgewählte Metallkomplexverbindungen gemäß den allgemeinen Formeln [M₂(R-HPTB)X₃] und [M'₄OX₆L] umfasst.

Hierbei handelt es sich bei den Metallatomen M um zwei jeweils unabhängig voneinander ausgewählte Metalle aus der Liste umfassend Fe, Co, Ni. Bei den Metallatomen M' handelt es sich um ein Metall, ausgewählt aus der Liste umfassend Cu, Ag, Au. Bei den Resten R handelt es sich um unabhängig voneinander ausgewählte Reste aus der Liste umfassend H, Methyl, Ethyl, Propyl, Benzyl, 2-Methyl-Pyridyl. Bei X handelt es sich um Halogenatome, unabhängig voneinander ausgewählt aus der Liste umfassend Cl, Br. Bei den Liganden L handelt es sich um stickstoffhaltige Liganden, unabhängig voneinander ausgewählt aus der Liste umfassend aliphatische Amine, aromatische Amine, Methylaminobenzol sowie substituierte Methylaminobenzole.

### Ausführungsbeispiele

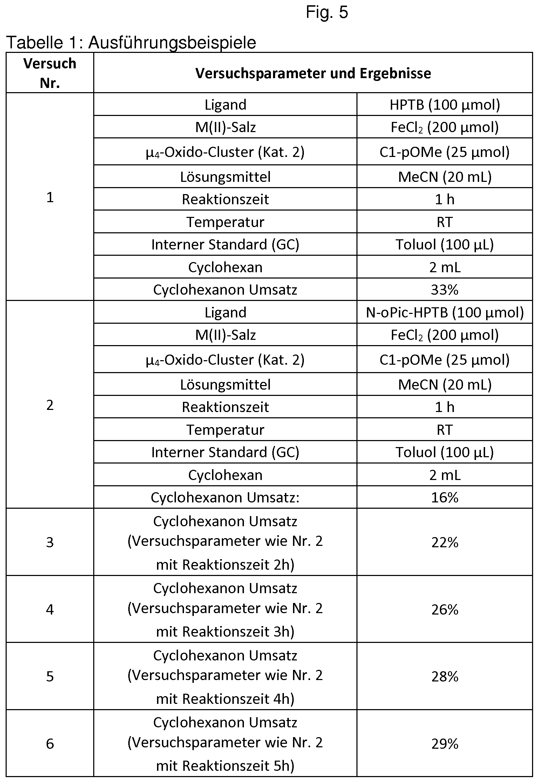

Im folgenden wird eine allgemeine Vorschrift zur Durchführung des erfindungsgemäßen Verfahrens offenbart; die detaillierten Versuchsparameter sowie die Versuchsergebnisse konkreter Beispiele sind in Tabelle 1 (Fig. 5) zusammengefasst. Dem Fachmann ist bekannt, dass die Wahl des Lösungsmittels oder Lösungsmittelgemisches in weiten Grenzen variiert werden kann, ohne den Umfang der Erfindung sowie ihrer Äquivalente zu verlassen. Beispielsweise fällt es in den Umfang der Erfindung, als Lösungsmittel neben Methanol oder Acetonitril beliebige weitere polare protische oder aprotische Lösungsmittel zu verwenden, die es gestatten, das Edukt sowie die Katalysatormischung unter den gewählten Reaktionsbedingungen zu lösen.

### Allgemeine Arbeitsvorschrift:

100 µmol Kat 1 werden in 10 mL Methanol, Acetonitril oder einem anderen geeigneten polaren Lösungsmittel gelöst. Alternativ kann Kat. 1 auch durch Zusammengeben von Ligand und Metallsalz (MX₂) im Lösungsmittel frisch erzeugt werden. Anschließend wird das Edukt, beispielsweise Cyclohexan (1 mL) zugegeben. Die Reaktionslösung bzw. Reaktionsmischung wird sodann, beispielsweise durch Behandlung mit Sauerstoff, aktiviert. Je nach gewünschter Aktivität des Katalysators 1, bzw. vorliegender Reaktivität des Katalysator/Edukt-Komplexes kann die Aktivierung mit reinem Sauerstoff oder einer Gasmischung, die neben Sauerstoff noch weitere Bestandteile, z. B. Schutzgase wie etwa Stickstoff, Helium, Argon oder Kohlendioxid enthalten kann, erfolgen. Nach erfolgter Aktivierung werden 25 µmol Kat 2 hinzugegeben und die Reaktionslösung im Sauerstoffstrom für 30 Minuten bei Raumtemperatur (RT) oder einer anderen gewünschten Reaktionstemperatur gerührt. Anschließend wird die Reaktionslösung in allgemeiner, dem Fachmann bekannter Art und Weise aufgearbeitet und das Produkt gereinigt, sofern dies erforderlich ist. Die direkte Analyse der Reaktionslösung kann, beispielsweise zur Überwachung des Reaktionsfortschritts, beispielsweise mittels GC nach vorheriger Kalibrierung mit Toluol als internem Standard erfolgen. Dabei zeigt sich, dass ausschließlich die Bildung von Cyclohexanon eintritt; es werden Ausbeuten von 60 % erzielt.

### Abbildungslegende

- Fig. 1: Beispiele erfindungsgemäßer HPTB-Liganden des Katalysators 1
- Fig. 2: Allgemeine Struktur des Katalysators 2
- Fig. 3: Beispiele für erfindungsgemäße Liganden L des Katalysators 2.
- Fig. 4: Beispiele von erfindungsgemäß verwendbaren Edukten
- Fig. 5: Tabellarische Zusammenstellung einzelner ausgewählter Ausführungsbeispiele (Tabelle 1)
- Fig. 6: Gaschromatogramm der prozessierten Reaktionsmischung bzw. Reaktionslösung von Versuch 2 gemäß Tabelle 1
- Fig. 7: Gaschromatogramm der prozessierten Reaktionsmischung bzw. Reaktionslösung von Versuch 3 gemäß Tabelle 1
- Fig. 8: Gaschromatogramm der prozessierten Reaktionsmischung bzw. Reaktionslösung von Versuch 4 gemäß Tabelle 1
- Fig. 9: Gaschromatogramm der prozessierten Reaktionsmischung bzw. Reaktionslösung von Versuch 5 gemäß Tabelle 1
- Fig. 10: Gaschromatogramm der prozessierten Reaktionsmischung bzw. Reaktionslösung von Versuch 6 gemäß Tabelle 1

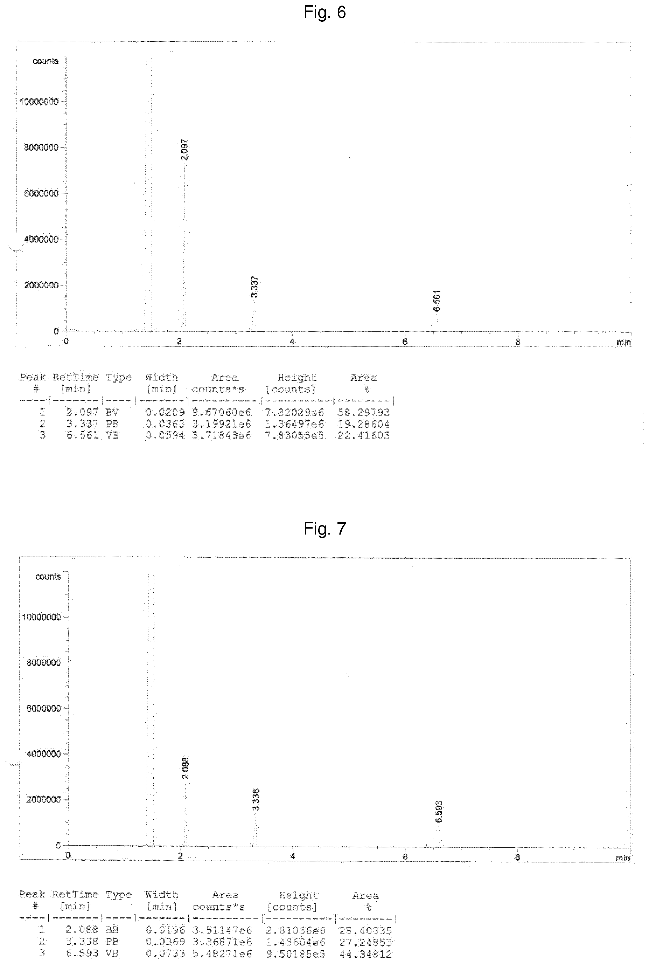

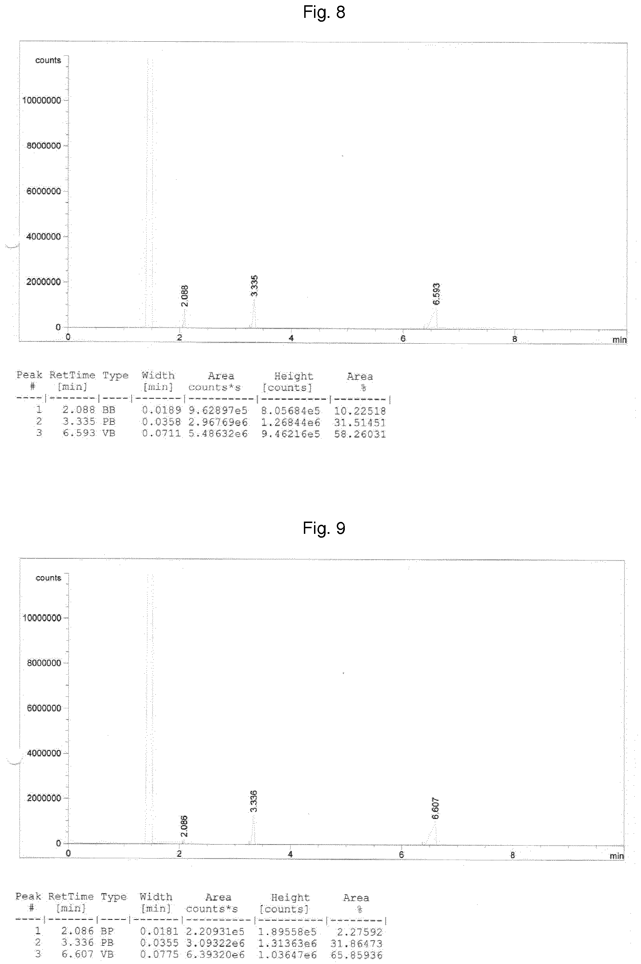

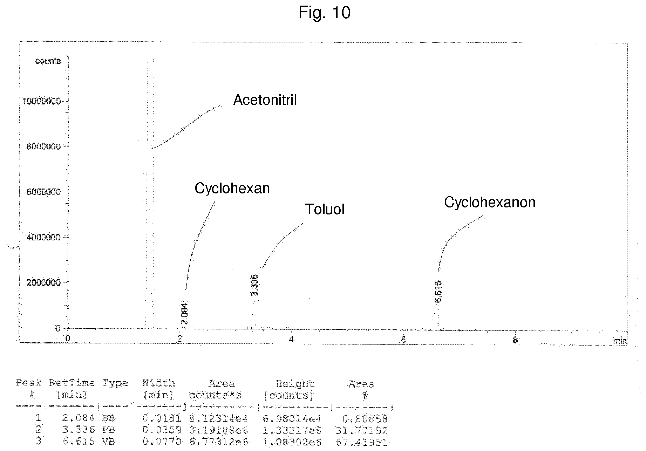

Bei den in den Abbildungen der Gaschromatogrammen angegebenen Auswertungsparametern (vgl. Fig. 6 bis Fig. 10) handelt es sich um die von dem Gaschromatographen direkt ausgegebenen Werte. Dem Fachmann ist deren Bedeutung bekannt. In Fig. 10 sind - beispielhaft für die Abbildungen Fig. 6 bis Fig. 10 die einzelnen Signale im Chromatogramm gekennzeichnet.

## Patentansprüche

1. Verfahren zur Oxidation von Alkanen zu Ketonen oder Aldehyden unter Verwendung von Sauerstoff als Oxidationsmittel, **dadurch gekennzeichnet dass** mindestens zwei unabhängig voneinander ausgewählte Katalysatoren gemäß den allgemeinen Formeln I (Katalysator 1),
[M₂(R-HPTB)X₃] (I),
und II (Katalysator 2),
[M'₄OX₆L] (II),
nacheinander oder gleichzeitig verwendet werden, um die Oxidationsreaktion zu katalysieren, wobei R-HPTB mehrzähnige N,N,N',N'-tetrakis(2-R-benzimidazolyl-methyl)2-hydroxy-1,3-diaminopropan-Liganden bezeichnet, und es sich bei den Metallatomen M um zwei jeweils unabhängig voneinander ausgewählte Metalle aus der Liste umfassend Fe, Co, Ni handelt, und es sich bei den Metallatomen M' um Atome eines Metalls handelt, ausgewählt aus der Liste umfassend Cu, Ag, Au, und es sich bei den Resten R um Reste handelt, unabhängig voneinander ausgewählt aus der Liste umfassend H, Methyl, Ethyl, Propyl, Benzyl, 2-Methyl-Pyridyl, und es sich bei X um Halogenatome handelt, unabhängig voneinander ausgewählt aus der Liste umfassend Cl, Br, und es sich bei den Liganden L um stickstoffhaltige Liganden handelt, unabhängig voneinander ausgewählt aus der Liste umfassend aliphatische Amine, aromatische Amine, Methylaminobenzol sowie substituierte Methylaminobenzole, wobei das Verfahren je nach Reaktivität des Edukts sowie von Katalysator 1 und Katalysator 2 in Gegenwart oder in Abwesenheit eines Lösungsmittels oder Lösungsmittelgemisches durchgeführt wird.

2. Verfahren gemäß Anspruch 1, **dadurch gekennzeichnet, dass** entweder
- Katalysator 1 zunächst mit dem zu oxidierenden Alkan umgesetzt und anschließend Katalysator 2 hinzugefügt wird, oder
- Katalysator 2 zunächst mit dem zu oxidierenden Alkan umgesetzt und anschließend Katalysator 1 hinzugefügt wird, oder
- Katalysator 1 und Katalysator 2 zunächst miteinander vermischt und anschließend das zu oxidierende Alkan hinzugefügt und umgesetzt wird.

3. Verfahren gemäß Anspruch 2, **dadurch gekennzeichnet, dass** zwischen den Zugaben von Katalysator 1, Katalysator 2 sowie dem zu oxidierenden Alkan mindestens ein Aktivierungsschritt zwischengeschaltet wird, um die Reaktivität der Reaktionsmischung in der jeweils vorliegenden Zusammensetzung zu verbessern, wobei es sich bei dem Aktivierungsschritt um Rühren und/oder Heizen und/oder Kühlen und/oder Begasung mit einem Gas und/oder Belichtung und/oder Beschallung mittels Ultraschall und/oder Zugabe weiterer Reagenzien handelt.

4. Verfahren gemäß einem der Ansprüche 1 bis 3, **dadurch gekennzeichnet, dass** das Oxidationsmittel Sauerstoff gasförmig in einer Konzentration von 1% bis 100% zugeführt wird.

5. Verfahren gemäß Anspruch 4, **dadurch gekennzeichnet, dass** das Oxidationsmittel Sauerstoff in einer Konzentration von 20% bis 25% zugeführt wird.

6. Verfahren gemäß Anspruch 4, **dadurch gekennzeichnet, dass** das Oxidationsmittel Sauerstoff in einer Konzentration von 80% bis 100% zugeführt wird.

7. Verfahren gemäß einem der Ansprüche 1 bis 6, **dadurch gekennzeichnet, dass** das Mischungsverhältnis von Katalysator 1 zu Katalysator 2 im Bereich von 1:100 bis 100:1 liegt.

8. Verfahren gemäß Anspruch 7, **dadurch gekennzeichnet, dass** das Mischungsverhältnis von Katalysator 1 zu Katalysator 2 im Bereich von 100:20 bis 100:30 liegt.

9. Verfahren gemäß einem der Ansprüche 1 bis 8, **dadurch gekennzeichnet, dass** das Verfahren bei einem Absolutdruck von 10² Pascal bis 10⁸ Pascal durchgeführt wird.

10. Verfahren gemäß Anspruch 9, **dadurch gekennzeichnet, dass** das Verfahren bei einem Absolutdruck von 10⁴ Pascal bis 10⁶ Pascal durchgeführt wird.

11. Verfahren gemäß einem der Ansprüche 1 bis 10, **dadurch gekennzeichnet, dass** das Verfahren bei einer Temperatur im Bereich von -80 °C bis +300 °C durchgeführt wird.

12. Verfahren gemäß Anspruch 11, **dadurch gekennzeichnet, dass** das Verfahren bei einer Temperatur im Bereich von -10 °C bis +100 °C durchgeführt wird.

13. Verfahren gemäß einem der Ansprüche 1 bis 12, **dadurch gekennzeichnet, dass** das Verfahren unter Bestrahlung mit elektromagnetischen Wellen der Wellenlängenbereiche des UV-, des sichtbaren- oder des Mikrowellenbereichs durchgeführt wird.

14. Verfahren gemäß einem der Ansprüche 1 bis 13, **dadurch gekennzeichnet, dass** das Verfahren in der Gasphase durchgeführt wird, wobei Katalysator 1 und Katalysator 2 auf einer geeigneten Oberfläche immobilisiert sind, und sowohl das Oxidationsmittel als auch das zu oxidierende Alkan gasförmig mit Katalysator 1 und Katalysator 2 in Kontakt gebracht und umgesetzt werden.

15. Verfahren gemäß einem der Ansprüche 1 bis 14, **dadurch gekennzeichnet, dass** das zu oxidierende Alkan ausgewählt ist aus der Liste umfassend Cyclopentan, Cyclohexan, Cycloheptan, Cyclooctan, Cycloalkane, Methan, Ethan, Propan, Butan, Pentan, Hexan, Heptan, Octan, Alkane, Toluol, o-Xylol, m-Xylol, p-Xylol, Ethylbenzol, Propylbenzol, aromatische Verbindungen mit Alkylseitenketten, Adamantan, Kuban, Käfigverbindungen.

16. Katalysatormischung zur Oxidation von Alkanen unter Verwendung von Sauerstoff als Oxidationsmittel, umfassend mindestens zwei unabhängig voneinander ausgewählte Metallkomplexverbindungen gemäß den allgemeinen Formeln I,
[M₂(R-HPTB)X₃] (I),
und II,
[M'₄OX₆L] (II),
wobei R-HPTB mehrzähnige N,N,N',N'-tetrakis(2-R-benzimidazolylmethyl)2-hydroxy-1,3-diaminopropan-Liganden bezeichnet, und es sich bei den Metallatomen M um zwei jeweils unabhängig voneinander ausgewählte Metalle aus der Liste umfassend Fe, Co, Ni handelt, und es sich bei den Metallatomen M' um ein Metall handelt, ausgewählt aus der Liste umfassend Cu, Ag, Au, und es sich bei den Resten R um Reste handelt, unabhängig voneinander ausgewählt aus der Liste umfassend H, Methyl, Ethyl, Propyl, Benzyl, 2-Methyl-Pyridyl, und es sich bei X um Halogenatome handelt, unabhängig voneinander ausgewählt aus der Liste umfassend Cl, Br, und es sich bei den Liganden L um stickstoffhaltige Liganden handelt, unabhängig voneinander ausgewählt aus der Liste umfassend aliphatische Amine, aromatische Amine, Methylaminobenzol sowie substituierte Methylaminobenzole.

## Claims

1. Process for oxidation of alkanes to ketones or aldehydes using oxygen as oxidising agent, **characterised in that** at least two catalysts according to general formula I (catalyst 1),
[M₂(R-HPTB)X₃] (I),
and general formula II (catalyst 2)
[M'₄OX₆L] (II),
both catalysts being chosen independently from each other, are applied sequentially or at the same time in order to catalyse the oxidation-reaction, whereat R-HPTB denotes multidentate N,N,N',N'-tetrakis(2-R-benzimidazolylmethyl)2-hydroxy-1,3-diaminopropane ligands and metal atoms M are respectively independently from each other chosen from the list comprising Fe, Co, Ni, and metal atoms M' are atoms of a metal chosen from the list comprising Cu, Ag, Au, and substituents R are independently from each other chosen from the list comprising H, methyl, ethyl, propyl, benzyl, 2-methyl-pyridyl, and X are halogen atoms, independently from each other chosen from the list comprising CI, Br, and ligands L are nitrogen-containing ligands, independently from each other chosen from the list comprising aliphatic amines, aromatic amines, methylaminobenzene as well as substituted methylaminobenzenes, whereat the process is performed in the presence or absence of a solvent or a mixture of solvents, depending on the reactivity of the educts as well as the reactivitiy of catalyst 1 and catalyst 2.

2. Process according to claim 1, **characterised in that** either
- catalyst 1 is at first reacted with the alkane to be oxidized and then catalyst 2 is being added , or
- catalyst 2 is at first reacted with the alkane to be oxidized and then catalyst 1 is being added , or
- catalyst 1 and catalyst 2 are admixed at first with each other and then the alkane to be oxidized is being added and reacted.

3. Process according to claim 2, **characterised in that** between the additions of catalyst 1, catalyst 2 as well as the alkane to be oxidized at least one intermediate step of activation is performed in order to improve the reactivity of the reaction mixture of the composition prevailing at that time, whereat the step of activation is stirring and/or heating and/or cooling and/or gassing with a gas and/or luminous exposure and/or exposure to ultrasound and/or addition of further reagents.

4. Process according to one of the claims 1 through 3, **characterised in that** the oxidsing agent oxygen is applied in gaseous form in a concentration within the range of 1 per cent until 100 per cent.

5. Process according to claim 4, **characterised in that** the oxidsing agent oxygen is applied in a concentration within the range of 20 per cent until 25 per cent.

6. Process according to claim 4, **characterised in that** the oxidsing agent oxygen is applied in a concentration within the range of 80 per cent until 100 per cent.

7. Process according to one of the claims 1 through 6, **characterised in that** the mixing ratio of catalyst 1 to catalyst 2 lies within the range from 1:100 until 100:1.

8. Process according to claim 7, **characterised in that** the mixing ratio of catalyst 1 to catalyst 2 lies within the range from 100:20 until 100:30.

9. Process according to one of the claims 1 through 8, **characterised in that** the process is performed at an absolute pressure ranging from 10² Pascal until 10⁸ Pascal.

10. Process according to claim 9, **characterised in that** the process is performed at an absolute pressure ranging from 10⁴ Pascal until 10⁶ Pascal.

11. Process according to one of the claims 1 through 10, **characterised in that** the process is performed at a temperature within the range from -80 °C until +300 °C.

12. Process according to claim 11, **characterised in that** the process is performed at a temperature within the range from -10 °C until +100 °C.

13. Process according to one of the claims 1 through 12, **characterised in that** the process is performed by irradiation with electromagnetic waves of a wavelength range of the uv-, visible- or the microwave-range.

14. Process according to one of the claims 1 through 13, **characterised in that** the process is performed in gaseous phase, whereat catalyst 1 and catalyst 2 are immobilised on a suitable surface and the oxidising agent as well as the alkane to be oxidized are brought into contact with and are reacted with catalyst 1 and catalyst 2 in gaseous phase.

15. Process according to one of the claims 1 through 14, **characterised in that** the alkane to be oxidized is chosen from the list comprising cyclopentane, cyclohexane, cycloheptane, cyclooctane, cycloalkane, methane, ethane, propane, butane, pentane, hexane, heptane, octane, alkanes, toluene, o-xylene, m-xylene, p-xylene, ethylbenzene, propylbenzene, aromatic compounds with alkyl side chains, adamantane, cubane, cage compounds.

16. Catalyst mixture for the oxidation of alkanes using oxygen as oxidising agent, comprising at least two metal complex compounds according to general formula I (catalyst 1),
[M₂(R-HPTB)X₃] (I),
and general formula II (catalyst 2),
[M'₄OX₆L] (II),
both catalysts being chosen independently from each other, whereat R-HPTB denotes multidentate N,N,N',N'-tetrakis(2-R-benzimidazolylmethyl)2-hydroxy-1,3-diaminopropane ligands and metal atoms M are respectively independently from each other chosen from the list comprising Fe, Co, Ni, and metal atoms M' are atoms of a metal chosen from the list comprising Cu, Ag, Au, and substituents R are independently from each other chosen from the list comprising H, methyl, ethyl, propyl, benzyl, 2-methyl-pyridyl, and X are halogen atoms, independently from each other chosen from the list comprising CI, Br, and ligands L are nitrogen-containing ligands, independently from each other chosen from the list comprising aliphatic amines, aromatic amines, methylaminobenzene as well as substituted methylaminobenzenes.

## Revendications

1. Procédé d'oxydation d'alcanes en cétones ou aldéhydes en utilisant de l'oxygène comme agent oxydant, **caractérisé en ce que** au moins deux catalyseurs choisis indépendamment selon les formules générales I (catalyseur 1),
[M₂(R-HPTB)X₃] (I),
et II (catalyseur 2),
[M'₄OX₆L] (II),
sont utilisés séquentiellement ou simultanément pour catalyser la réaction d'oxydation, où R-HPTB désigne des ligands multidentaires N,N,N',N'-tétrakis(2-R-benzimidazolyl-méthyl)2-hydroxy-1,3-diaminopropane, et les atomes de métal M sont deux métaux choisis chacun indépendamment dans la liste comprenant Fe, Co, Ni et les atomes de métal M' sont des atomes d'un métal choisi dans la liste comprenant Cu, Ag, Au, et les radicaux R sont des radicaux choisis indépendamment dans la liste comprenant H, méthyle, éthyle, propyle, benzyle, 2-méthyl-pyridyle, et X sont des atomes d'halogène choisis indépendamment dans la liste comprenant Cl, Br, et les ligands L sont des ligands contenant de l'azote choisis indépendamment dans la liste comprenant les amines aliphatiques, amines aromatiques, méthylaminobenzène et méthylaminobenzènes substitués, le procédé étant réalisé en présence ou en l'absence d'un solvant ou d'un mélange de solvants, en fonction de la réactivité de la matière de départ et du catalyseur 1 et du catalyseur.

2. Procédé selon la revendication 1, **caractérisé en ce que** soit
- Catalyseur 1 d'abord avec l'alcane à oxyder puis le catalyseur 2 est ajouté, ou
- Catalyseur 2 d'abord avec l'alcane à oxyder puis le catalyseur 1 est ajouté, ou
- le catalyseur 1 et le catalyseur 2 sont d'abord mélangés et on ajoute ensuite l'alcane à oxyder et est mis en œuvre.

3. Procédé selon la revendication 2, **caractérisé en ce qu'**au moins une étape d'activation est intercalée entre les additions de catalyseur 1, de catalyseur 2 et de l'alcane à oxyder afin d'améliorer la réactivité du mélange réactionnel dans la composition respective présente, l'étape d'activation étant l'agitation et/ou le chauffage et/ou le refroidissement et/ou l'aération avec un gaz et/ou l'exposition et/ou la sonication au moyen d'ultrasons et/ou l'addition d'autres réactifs.

4. Procédé selon l'une quelconque des revendications 1 à 3, **caractérisé en ce que** l'agent oxydant oxygène est fourni sous forme gazeuse à une concentration de 1 % à 100 %.

5. Procédé selon la revendication 4, **caractérisé en ce que** l'agent oxydant oxygène est fourni dans une concentration de 20 à 25 %.

6. Procédé selon la revendication 4, **caractérisé en ce que** l'agent oxydant oxygène est fourni dans une concentration de 80% à 100%.

7. Procédé selon l'une quelconque des revendications 1 à 6, **caractérisé en ce que** le rapport de mélange du catalyseur 1 au catalyseur 2 se situe dans la plage de 1:100 à 100:1.

8. Procédé selon la revendication 7, **caractérisé en ce que** le rapport de mélange du catalyseur 1 au catalyseur 2 se situe dans la plage de 100:20 à 100:30.

9. Procédé selon l'une des revendications 1 à 8, **caractérisé en ce que** le processus est réalisé à une pression absolue de 10² Pascal à 10⁸ Pascal.

10. Procédé selon la revendication 9, **caractérisé en ce que** le processus est réalisé à une pression absolue de 10⁴ Pascal à 10⁶ Pascal.

11. Procédé selon l'une quelconque des revendications 1 à 10, **caractérisé en ce que** le procédé est réalisé à une température comprise entre -80 °C et +300 °C.

12. Procédé selon la revendication 11, **caractérisé en ce que** le procédé est effectué à une température comprise entre -10 °C et +100 °C.

13. Procédé selon l'une quelconque des revendications 1 à 12, **caractérisé en ce que** le procédé est réalisé sous irradiation avec des ondes électromagnétiques des gammes de longueurs d'onde de l'UV, du visible ou des micro-ondes.

14. Procédé selon l'une quelconque des revendications 1 à 13, **caractérisé en ce que** le procédé est mis en oeuvre en phase gazeuse, le catalyseur 1 et le catalyseur 2 étant immobilisés sur une surface appropriée, et l'agent oxydant ainsi que l'alcane à oxyder étant mis en contact avec le catalyseur 1 et le catalyseur 2 sous forme gazeuse et mis à réagir.

15. Procédé selon l'une quelconque des revendications 1 à 14, **caractérisé en ce que** l'alcane à oxyder est choisi dans la liste comprenant le cyclopentane, le cyclohexane, le cycloheptane, le cyclooctane, les cycloalcanes, le méthane, éthane, propane, butane, pentane, hexane, heptane, octane, alcanes, toluène, o-xylène, m-xylène, p-xylène, éthylbenzène, propylbenzène, composés aromatiques à chaînes latérales alkyles, adamantane, cubane, composés en cage.

16. Mélange de catalyseurs pour l'oxydation d'alcanes en utilisant de l'oxygène comme agent oxydant, comprenant au moins deux composés complexes métalliques choisis indépendamment l'un de l'autre de formules générales I,
[M₂(R-HPTB)X₃] (I),
et II,
[M'₄OX₆L] (II),
dans laquelle R-HPTB désigne des ligands N,N,N',N'-tétrakis(2-R-benzimidazolyl-méthyl)2-hydroxy-1,3-diaminopropane multidentés, et les atomes métalliques M sont deux métaux choisis indépendamment dans la liste comprenant Fe, Co, Ni, et les atomes métalliques M' sont un métal choisi dans la liste comprenant Cu, Ag, Au, et les radicaux R sont des radicaux, choisis indépendamment dans la liste comprenant H, méthyle, éthyle, propyle, benzyle, 2-méthyl-pyridyle, et X sont des atomes d'halogène choisis indépendamment dans la liste comprenant Cl, Br, et les ligands L sont des ligands contenant de l'azote choisis indépendamment dans la liste comprenant les amines aliphatiques, les amines aromatiques, le méthylaminobenzène et les méthylaminobenzènes substitués.
